(19) Europäisches Patentamt
European Patent Office
Office européen des brevets

(11) Veröffentlichungsnummer: **0 001 618**
**A2**

# (12) EUROPÄISCHE PATENTANMELDUNG

(21) Anmeldenummer: 78101134.1

(22) Anmeldetag: 13.10.78

(51) Int. Cl.$^2$: C 07 D 403/06
A 61 K 31/55
//C07D241/52, A61K31/495

(30) Priorität: 25.10.77 DE 2747700

(43) Veröffentlichungstag der Anmeldung: 02.05.79 Patentblatt 79/9

(84) Benannte Vertragsstaaten: BE CH DE FR GB NL

(71) Anmelder: Bayer Aktiengesellschaft
Zentralbereich Patente,Marken und Lizenzen Bayerwerk
D-5090 Leverkusen 1(DE)

(72) Erfinder: Seng, Florin, Dr.
Am Katterbach 46
D-5060 Bergisch-Gladbach 2(DE)

(72) Erfinder: Metzger, Karl Georg, Dr.
Pahlkestrasse 15
D-5600 Wuppertal 1(DE)

## (54) Verfahren zur Herstellung von neuen Chinoxalin-di-N-oxiden sowie ihre Verwendung als Arzneimittel.

(57) Die vorliegende Erfindung betrifft ein neues chemisch eigenartiges Verfahren zur Herstellung von neuen antibakteriell wirksamen Chinoxalin-di-N- oxiden sowie ihre Verwendung als Arzneimittel, insbesondere als antibakterielle Mittel und als Futterzusatzmittel.

Die neuen Chinoxalin-di-N-oxide weisen starke antibakterielle Eigenschaften auf und werden Menschen oder Tieren appliziert, die an bakteriellen Erkrankungen leiden.

EP 0 001 618 A2

BAYER AKTIENGESELLSCHAFT
Zentralbereich
Patente, Marken und Lizenzen
5090 Leverkusen, Bayerwerk
Si-kl
IVb/(Pha)

**Verfahren zur Herstellung von neuen Chinoxalin-di-N-oxiden sowie ihre Verwendung als Arzneimittel**

Die vorliegende Erfindung betrifft ein neues chemisch eigenartiges Verfahren zur Herstellung von neuen antibakteriell wirksamen Chinoxalin-di-N-oxiden sowie ihre Verwendung als Arzneimittel, insbesondere als antibakterille Mittel, und als Futterzusatzmittel.

Es ist bereits bekannt geworden, daß 2-Methyl-3-carbomoyl-chinoxalin-di-N-oxide antibakterielle Eigenschaften besitzen [J.K. Landquist und J.A. Silk, J.Chem.Soc. 1956, 2052]. Diese Stoffe sind jedoch toxisch und bezüglich ihrer Löslichkeit unbefriedigend.

Es wurde gefunden, daß man die neuen Chinoxalin-di-N-oxide der Formel (I)

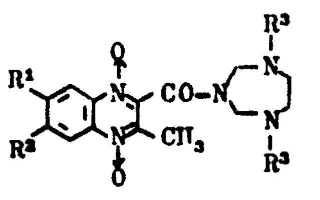 (I)

in welcher

$R^1$ und $R^2$ gleich oder verschieden sein können und für Wasserstoff, Halogen, Alkyl oder Alkoxy stehen und

$R^3$ für Alkyl steht

erhält, wenn man 2-Methyl-3-carbamoyl-chinoxalin-di-N-oxide der Formel (II)

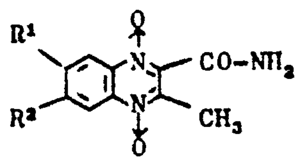

(II)

in welcher
$R^1$ und $R^2$ die oben angegebene Bedeutung haben,
mit Formaldehyd oder formaldehydabgebenden Mitteln und Imidazolidinen der Formel (III)

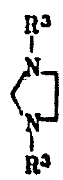

(III)

in welcher
$R^3$ die oben angegebene Bedeutung hat,

in Gegenwart von inerten Lösungsmitteln bei Temperaturen zwischen 0° und 100° C umsetzt.

Die neuen Chinoxalin-di-N-oxide der Formel (I) weisen starke antibakterielle Eigenschaften auf.

Es ist als ausgesprochen überraschend zu bezeichnen, daß gemäß der erfindungsgemäßen Umsetzung Chinoxalin-di-N-oxide der Formel (I) entstehen. Im Hinblick auf den Stand der Technik mußte man nämlich im Falle der Umsetzung von Formaldehyd und Carbamoylverbindungen der Formel (II) in Gegenwart von basischen Katalysatoren erwarten, daß die entsprechenden Hydroxymethylverbindungen entstehen [Angew. Chem. 69, 463 (1957)].

Daß die hierbei als basische Katalysatoren verwendeten Imidazolidine der Formel (III) mit in die Reaktion einbezogen werden, war nicht vorhersehbar.

Verwendet man 2-Methyl-3-carbamoyl-chinoxalin-di-N-oxid, Formaldehyd und 1,3-Dimethyl-imidazolidin als Ausgangsstoffe, so kann der Reaktionsablauf durch das folgende Formelschema wiedergegeben werden:

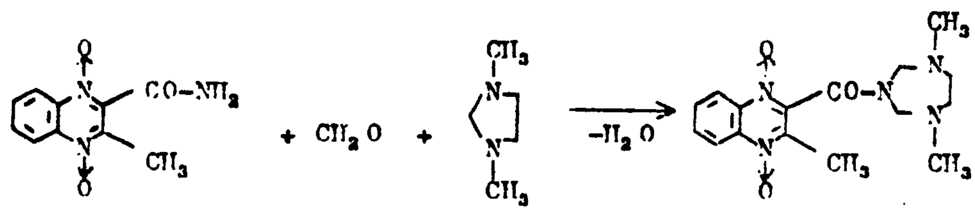

In der Formel (I) stehen $R^1$ und $R^2$ für Wasserstoff, Halogen wie z.B. Fluor, Chlor und Brom, insbesondere Fluor und Chlor, Alkylreste mit 1 - 4 Kohlenstoffatomen oder Alkoxyreste mit 1 - 4 Kohlenstoffatomen, wobei einer der Reste $R^1$ und $R^2$ immer Wasserstoff ist. $R^3$ steht für einen gesättigten aliphatischen Kohlenwasserstoffrest mit 1 - 4 Kohlenstoffatomen. Die erfindungsgemäß verwendbaren 2-Methyl-3-carbamoyl-chinoxalin-di-N-oxide sind bekannt /J.K. Landquist und J.A. Silk, J.Chem.Soc. 1956, 2052/.
Als Beispiele seien genannt:

2-Methyl-3-carbamoyl-chinoxalin-di-N-oxid
2-Methyl-3-carbamoyl-5 bzw.6-methyl-chinoxalin-di-N-oxid
2-Methyl-3-carbamoyl-äthyl-chinoxalin-di-N-oxid
2-Methyl-3-carbamoyl-propyl-chinoxalin-di-N-oxid
2-Methyl-3-carbamoyl-fluor-chinoxalin-di-N-oxid
2-Methyl-3-carbamoyl-chlor-chinoxalin-di-N-oxid
2-Methyl-3-carbamoyl-brom-chinoxalin-di-N-oxid
2-Methyl-3-carbamoyl-methoxy-chinoxalin-di-N-oxid
2-Methyl-3-carbamoyl-äthoxy-chinoxalin-di-N-oxid

Die erfindungsgemäß verwendbaren Imidazolidine sind ebenfalls bekannt. Sie werden durch Umsetzung von Äthylendiaminen mit Formaldehyd erhalten [The Chemistry of Heterocyclic Compounds, Imidazole, Interscience Publishers INC, New York, Seite 242].

Formaldehyd kann in fester Form als Paraformaldehyd oder Trioxan oder in Form einer wässrigen Lösung verwendet werden.

Als Verdünnungsmittel kommen polare organische Lösungsmittel in Frage. Hierzu gehören vorzugsweise Nitrile wie Acetonitril, Propionsäurenitril, Adipinsäuredinitril, Benzonitril, Formamide wie Formamid, N-Methylformamid, N,N-Dimethyl-formamid und Dimethylsulfoxid.

Die Reaktionstemperaturen können in einem größeren Bereich variiert werden. Im allgemeinen arbeitet man zwischen 0° und etwa 100° C, vorzugsweise zwischen 20° C und 70° C.

Bei der Durchführung des erfindungsgemäßen Verfahrens setzt man auf 1 Mol 2-Methyl-3-carbamoyl-chinoxalin-di-N-oxid mindestens 1 Mol Formaldehyd und mindestens 1 Mol Imidazolidin ein. Es kann aber auch ein Ueberschuß an Formaldehyd und Imidiazolidin verwendet werden.

Die Reaktion wird so durchgeführt, daß man das Gemisch aus Chinoxalin-di-N-oxid, Formaldehyd und Imidazolidin in einem Verdünnungsmittel bei Temperaturen zwischen 20° und 70° C umsetzt. Das Ende der Reaktion kann daran erkannt werden, daß sich die Endprodukte im Gegensatz zu den Ausgangsprodukten in wässrigen Säuren klar lösen. Die Reaktionsprodukte scheiden sich meist in fester kristalliner Form ab und können abgesaugt werden. In einigen Fällen sind die Endprodukte im Verdünnungsmittel gelöst und können durch Eindampfen der Reaktionslösung gewonnen werden.

Die neuen Wirkstoffe wiesen starke antibakterielle Eigenschaften auf:

Subkutane $ED_{100}$ x) bei infizierten Mäusen in mg/kg

| Substanz | mg/kg Körpergewicht Infektionskeim E. coli Neumann |
|---|---|
| Beispiel 1 | 5 - 10 |
| Beispiel 4 | 20 |
| Beispiel 2 | 10 |

x) Als $ED_{100}$ wird die Dosis bezeichnet, mit der 100 % der Tiere geheilt werden.

Die ausgezeichnete und breite antibakterielle Wirksamkeit der Wirkstoffe ermöglicht ihren Einsatz sowohl in der Human- als auch in der Veterinärmedizin, wobei sie sowohl zur Verhütung als auch zur Behandlung von systemischen oder lokalen bakteriellen Infektionen verwendet werden können.

Die Verbindungen können auch als Futterzusatzmittel zur Förderung des Wachstums und zur Verbesserung der Futterauswertung in der Tierhaltung, insbesondere bei der Haltung von Mastvieh, wie z. B. Rindern, Schweinen und Geflügel usw., verwendet werden.

Die Applikation der Wirkstoffe erfolgt dann vorzugsweise über das Futter und/oder Trinkwasser. Die Wirkstoffe können aber auch in Futterkonzentraten sowie in Vitamine und/oder Mineralsalze enthaltenden Zubereitungen verwendet werden.

Die Vermischung mit dem Futter oder in den Futterkonzentraten und den übrigen Futterzubereitungen erfolgt gegebenenfalls in Form eines Praemix nach den üblichen Methoden.

Zur vorliegenden Erfindung gehören pharmazeutische Zubereitungen, die neben nichttoxischen, inerten pharmazeutisch geeigneten Trägerstoffen eine oder mehrere Verbindungen der Formel (I) enthalten oder die aus einer oder mehreren Verbindungen der Formel (I) bestehen, sowie ein Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen, vorliegen, deren Wirkstoffgehalt einem Bruchteil oder einem Vielfachen einer Einzeldosis entsprechen. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoff(e) neben den üblichen Trägerstoffen enthalten, wie a) Füll- und Streckmittel, z. B. Stärken, Milchzucker, Rohrzucker, Glukose, Mannit und Kieselsäure, b) Bindemittel, z. B. Carboxymethylcellulose, Alginate, Gelatine, Polyvinylpyrrolidon, c) Feuchthaltemittel, z.B. Glycerin, d) Sprengmittel, z. B. Agar-Agar, Calciumcarbonat und Natriumbicarbonat, e) Lösungsverzögerer, z. B. Paraffin, und f) Resorptionsbeschleuniger, z. B. quarternäre Ammoniumverbindungen, g) Netzmittel, z. B. Cetylalkohol, Glycerinmonostearat, h) Adsorptionsmittel, z. B. Kaolin und Bentonit, und i) Gleitmittel, z. B. Talkum, Calcium- und Magnesiumstearat und feste Polyäthylenglykole oder Gemische der unter a) bis i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinal-

traktes gegebenenfalls verzögert werden, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z. B. Polyäthylenglykole, Fette, z. B. Kakaofett, und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilicat und Polyamidpulver oder Gemische dieser Stoffe. Sprays können zusätzlich die üblichen Treibmittel, z. B. Chlorfluorkohlenwasserstoffe, enthalten.

Lösungen und Emulsionen können neben dem oder den Wirkstoffen die üblichen Trägerstoffe, wie Lösungsmittel, Lösungsvermittler und Emulgatoren, z. B. Wasser, Äthylalkohol, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycium, Glycerinformal, Tetrahydrofurfurylalkohol, Polyäthylglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z. B. Wasser, Äthylalkohol, Propylenglykol, Suspendiermittel, z. B. äthoxylierte Isostearylalkohole, Polyoxyäthylensorbit- und -sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z. B. Pfefferminzöl und Eukalyptusöl, und Süßmittel, z. B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gewichtsprozenten der Gesamtmischung vorhanden sein.

Die oben aufgeführten pharmazeutischen Zubereitungen können außer Verbindungen der Formel (I) auch andere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Zur vorliegenden Erfindung gehört auch die Verwendung der Verbindungen der Formel (I) sowie von pharmazeutischen Zubereitungen, die eine oder mehrere Verbindungen der Formel (I) enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rectal, vorzugsweise parenteral, insbesondere intravenös, appliziert werden.

Die neuen Wirkstoffe sollen sowohl in der Human- als auch in der Veterinärmedizin zur Behandlung bakterieller Erkrankungen verwendet werden. Dabei hat es sich als vorteilhaft erwiesen, den oder die Wirkstoffe in Mengen von etwa 5 bis etwa 150, vorzugsweise 25 bis 75 mg/kg Körpergewicht je 24 Stunden, gegebenenfalls in Form mehrerer Einzelgaben zur Erzielung der gewünschten Ergebnisse zu verabreichen. Es kann jedoch erforderlich sein, von den genannten Dosierungen abzuweichen, und zwar in Abhängigkeit von der Art und dem Körpergewicht des zu behandelnden Objektes, der Art und der

Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann  aufgrund seines Fachwissens leicht erfolgen.

Beispiel 1

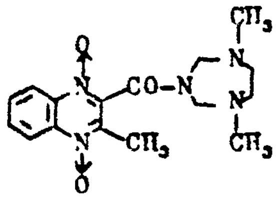

21,9 g (0,1 Mol) 2-Methyl-3-carbamoylchinoxalin-di-N-oxid werden in 200 ml Acetonitril suspendiert, mit 3 g (0,1 Mol) Paraformaldehyd und 12 g (0,12 Mol) 1,3-Dimethylimidazolidin versetzt und auf 70° C erwärmt. Nach einer Stunde ist alles gelöst. Die Lösung wird heiß filtriert und anschließend mit Eis abgekühlt. Dabei fallen 21 g (63,5 %) gelbe Kristalle aus, die bei 133 - 145° schmelzen.

Analyse $C_{16}H_{21}N_5O_3$ (331)

| | | | | | | |
|---|---|---|---|---|---|---|
| ber.: | C | 58,0 | H | 6,34 | N | 21,1 |
| gef.: | C | 58,0 | H | 6,5 | N | 21,0 |

Analog wurden erhalten:

2. 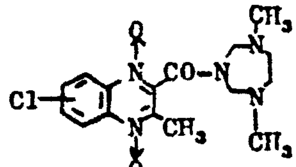 Fp: 190°

3. 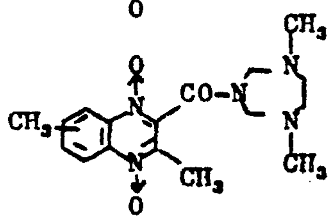 Fp: 162°

4. 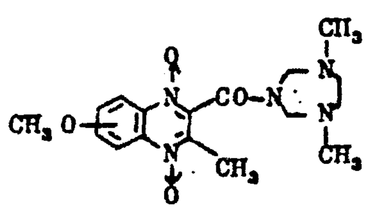 Fp: 150°

Patentansprüche

1. Verfahren zur Herstellung von Chinoxalin-di-N-oxiden der Formel (I)

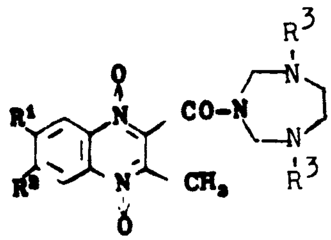

(I)

in welcher

$R^1$ und $R^2$ gleich oder verschieden sein können und für Wasserstoff, Halogen, Alkyl oder Alkoxy stehen und

$R^3$ für einen Alkylrest steht,

dadurch gekennzeichnet, daß man 2-Methyl-3-carbamoyl-chinoxalin-di-N-oxide der Formel (II)

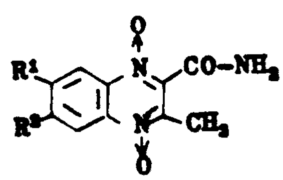

(II)

in welcher

$R^1$ und $R^2$ die oben angegebene Bedeutung haben

mit Formaldehyd oder formaldehydabgebenden Mitteln und Imidazolidinen der Formel (III)

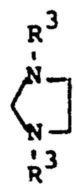

(III)

in welcher

$R^3$ die oben angegebene Bedeutung hat,

in Gegenwart von inerten Lösungsmitteln bei Temperaturen zwischen 0° und 100°C umsetzt.

2. Chinoxalin-di-N-oxide gemäß der Formel (I) in Anspruch 1.

3. Arzneimittel, gekennzeichnet durch einen Gehalt an mindestens einem Chinoxalin-di-N-oxid gemäß der Formel (I) in Anspruch 1.

4. Verfahren zur Herstellung von antibakteriellen Mitteln, dadurch gekennzeichnet, daß man Chinoxalin-di-N-oxide gemäß der Formel (I) in Anspruch 1 mit inerten, nichttoxischen, pharmazeutisch geeigneten Trägerstoffen vermischt.

5. Verfahren zur Behandlung von bakteriellen Erkrankungen, dadurch gekennzeichnet, daß man Chinoxalin-di-N-oxide gemäß der Formel (I) in Anspruch 1 Menschen oder Tieren appliziert, die an bakteriellen Erkrankungen leiden.